# EUROPÄISCHE PATENTSCHRIFT

(11) **EP 0 474 015 B1**
(45) Veröffentlichungstag und Bekanntmachung des Hinweises auf die Patenterteilung: **15.06.1994**
(21) Anmeldenummer: 91113877.4
(22) Anmeldetag: 19.08.1991
(51) Int. Cl.: A61F 2/36, A61F 2/38

(54) **Endoprothese**
Endoprosthesis
Endoprothèse

(30) Priorität: 07.09.1990 DE 4028510
(43) Veröffentlichungstag der Anmeldung: 11.03.1992
(73) Patentinhaber: Waldemar Link (GmbH & Co.), D-22315 Hamburg (DE)
(72) Erfinder: Keller, Arnold, W-2061 Kayhude (DE)
(74) Vertreter: Glawe, Delfs, Moll & Partner

(56) Entgegenhaltungen:
- EP-A- 0 000 549
- CH-A- 660 955
- FR-A- 1 594 365
- FR-A- 2 222 889
- US-A- 3 102 536

## Beschreibung

Die Erfindung betrifft eine Endoprothese mit zwei oder mehr Einzelteilen, von denen einer ein Konusteil mit einer konusförmigen Bohrung und von denen ein anderer ein Konusteil mit einem komplementären konusförmigen Vorsprung aufweist, wobei die Teile durch Einführen des konusförmigen Vorsprungs in die konusförmige Bohrung verbunden sind und im Bereich der Konusverbindung eine Sicherungsschraube vorgesehen ist, die in einem der Konusteile eingeschraubt ist und die mit ihrem vorderen Ende in eine Ausnehmung im anderen Konusteil eingreift.

Endoprothesen bestehen häufig aus zwei oder mehr Einzelteilen, die dann je nach Bedarf zusammengesetzt werden können, z.B. einem Prothesenhauptteil und einem Prothesenkopf. Solche mehrteiligen Prothesen haben den Vorteil, daß mit verhältnismäßig wenigen Einzelteilen sehr viele Kombinationen zwischen Prothesenkopf und Prothesenhauptteil hergestellt werden können, so daß die Zahl der vorrätig zu haltenden Teile geringer ist.

Die Prothese wird aus den beiden Einzelteilen dadurch zusammengesetzt, daß der Teil mit der konusförmigen Bohrung auf den Teil mit dem konusförmigen Vorsprung aufgesetzt und festgeschlagen wird. Hierdurch erhält man eine im allgemeinen zuverlässige und dauerhafte Verbindung, wie sie erforderlich ist.

Es ist bekannt, eine solche an sich schon feste Verbindung durch eine zusätzliche Sicherungsschraube zu sichern (EP-A-0 000 549).

Die Aufgabe der Erfindung besteht in der Schaffung einer Endoprothese der eingangs genannten Art, bei der die beiden Teile noch zuverlässiger dauerhaft miteinander verbunden werden können.

Die erfindungsgemäße Lösung besteht darin, daß die Achse (8) der Sicherungsschraube (6) im wesentlichen senkrecht zur konusachse steht und daß das vordere Ende der Sicherungsschraube kegel- oder kegelstumpfförmig ist und mit der Ausnehmung exzentrisch zusammenwirkt.

Durch das exzentrische Zusammenwirken sichert die Sicherungsschraube nicht nur die Verbindung, sondern übt eine zusätzliche Kraft in der Richtung aus, in der die Konusverbindung noch mehr zusammengedrückt wird. Eine solche exzentrisch wirkende Konusschraube ist zwar aus dem Regalbau bekannt (FR-A-2 222 889). Abgesehen davon, daß der Regalbau für den Konstrukteur einer Endoprothese keine Anregungen geben kann, hat dort die Konusschraube die Aufgabe, zwei ineinander verschiebbare Teile zusammenzuziehen, wobei die Verbindung ohne die Konusschraube auseinanderfällt. Bei der Erfindung ist die Konusverbindung für sich allein schon eine zuverlässige feste Verbindung, deren Zuverlässigkeit aber noch durch die erfindungsgemäße Anordnung der Konusschraube weiter erhöht wird.

Solche Sicherungsschrauben werden normalerweise nur dort verwendet, wo die beiden Teile ohne solche Schrauben nicht zuverlässig aneinander haften würden, z.B., wenn eine zylinderförmige Bohrung auf einen zylinderförmigen Halter aufgesetzt wird. Es ist das Verdienst der Erfindung erkannt zu haben, daß durch eine Sicherungsschraube, obwohl diese bei einer Konusverbindung doch gar nicht notwendig erscheint, eine noch sicherere Befestigung erhalten werden kann.

Bei einer vorteilhaften Ausführungsform ist die Sicherungsschraube im äußeren Konusteil angeordnet und wirkt mit einer Bohrung oder einer Ringnut im inneren Konusteil zusammen.

Ein wesentliches Merkmal ist, daß das vordere Ende der Sicherungsschraube mit der Ausnehmung exzentrisch oder achsenversetzt zusammenwirkt. Ist dabei die Sicherungsschraube im äußeren Konusteil angeordnet, so sollte das vordere Ende der Sicherungsschraube mit der Ausnehmung exzentrisch bzw. achsenversetzt auf der Seite der Schraubenachse zusammenwirken, die zum sich verjüngenden Konusende gerichtet ist.

Bei dieser exzentrischen Anordnung fixiert die Sicherungsschraube nicht nur die vorher durch Zusammenschlagen erreichte Stellung, sondern übt einseitig noch eine Kraft aus, durch die die beiden Konusteile noch weiter zusammengedrückt oder zusammengespannt werden. Hierdurch können z.B. Toleranzen ausgeglichen werden. Selbst bei sehr genau gearbeiteten Konusteilen passen möglicherweise Sicherungsschraube und Ausnehmung nicht genau konzentrisch aufeinander, da eine kleine Maßabweichung wegen der üblicherweise verhältnismäßig geringen Steigung des Konus bzw. eines normalerweise sehr kleinen Konuswinkels in Axialrichtung zu einer größeren Verschiebung führen kann. Solche Toleranzen können durch die exzentrische Anordnung ausgeglichen werden. Auch dann, wenn die Konusverbindung irrtümlich nicht fest genug hergestellt war oder aber infolge Alterungsprozessen die Konsuverbindung lose zu werden droht, so wird durch die Sicherungsschraube nicht ein erneutes festeres Zusammenpressen der Konusteile durch äußere Belastungen verhindert, da die Sicherungsschraube bzw. Sicherungsschrauben in dieser Richtung keine Kraft ausüben, sondern eine Bewegung gestatten. In den erwähnten ungünstigen Fällen wird also bei dieser Ausführungsform verhindert, daß die Konusschraube genau die gegenteilige Wirkung hat, nämlich eine festere Verbindung zu verhindern.

Ein weiterer Vorteil dieser Ausführungsform besteht darin, daß die Sicherungsschraube das Zusammensintern der Konusverbindung nicht behindert, sondern durch den Achsenversatz der Sicherungsschraube zur Ausnehmung bei Betätigung der Sicherungsschraube ein noch festeres Zusammenpressen der Konusverbindung in Längsrichtung bewirkt.

Bei einer anderen vorteilhaften Ausführungsform ist die Sicherungsschraube in einer mit Gewinde versehenen Bohrung des inneren Konusteils angeordnet und wirkt mit einer Bohrung oder einem sich über einen Teil des Umfangs erstreckenden Schlitz zusammen. Die Schraube kann dabei von außen mit einem Werkzeug gedreht werden, das durch den äußeren Konusteil hindurchgesteckt wird, indem die Bohrung im inneren Konusteil als Durchgangsbohrung ausgebildet ist und im äußeren Konusteil auf der radial gegenüberliegenden Seite der Ausnehmung, in die die Schraube eingreifen soll, eine Gegenbohrung oder ein entsprechender Schlitz vorgesehen ist. Man könnte aber auch vorsehen, daß die Schraube an ihrem vorderen Ende kegelstumpfförmig ausgebildet ist, wobei dann das Werkzeug an der flachen Vorderfläche des Kegelstumpfes angreift, das Werkzeug also an dem Ende der Schraube angreift, an dem die Schraube eine Kraft auf den anderen Konusteil ausüben soll.

Auch bei dieser Ausführungsform ist es wesentlich, wenn die Sicherungsschraube mit der Ausnehmung exzentrisch oder achsenversetzt zusammenwirkt und zwar auf der Seite der Schraubenachse, die vom sich verjüngenden Konusende weggerichtet ist. Es werden dann ebenfalls die oben im Zusammenhang mit der exzentrischen Anordnung beschriebenen besonderen Vorteile erhalten.

Wenn die Wände der Ausnehmung, mit denen die Sicherungsschraube zusammenwirkt, wenigstens im zur Sicherungsschraube weisenden Teil abgeschrägt sind, findet hier kein punktförmiger Kontakt statt, der unter Umständen zu einer Deformierung der Schraube oder der Wände führen könnte. Es können so größere Kräfte übertragen werden. Dabei sollte der Winkel der Abschrägung dem entsprechenden Winkel des kegel- oder kegelstumpfförmigen vorderen Endes der Sicherungsschraube möglichst genau entsprechen.

Statt eine einzelne Sicherungsschraube in die entsprechende mit Gewinde versehene Bohrung einzuschrauben, kann nach dem Festschrauben hier noch eine Konterschraube eingeschraubt und festgezogen werden.

Obwohl in vielen Fällen eine Sicherungsschraube ausreicht, kann auch mehr als eine Sicherungsschraube vorgesehen sein. Die beiden Sicherungsschrauben können dabei z.B. auf gegenüberliegenden Seiten des Konus wirken. Um das Lösen der Sicherungsschraube oder -schrauben zuverlässig zu verhindern, kann hinter der bzw. den Sicherungsschrauben eine Konterschraube eingeschraubt werden.

Die Erfindung wird im folgenden anhand von vorteilhaften Ausführungsformen unter Bezugnahme auf die beigefügten Zeichnungen beschrieben. Es zeigen:
- Fig. 1: teilweise im Querschnitt eine erste Ausführungsform der Erfindung;
- Fig. 2: teilweise im Querschnitt eine zweite Ausführungsform;
- Fig. 3: teilweise im Querschnitt eine dritte Ausführungsform; und
- Fig. 4: die Ausführungsform der Fig. 3 mit einer zusätzlichen Konterschraube.

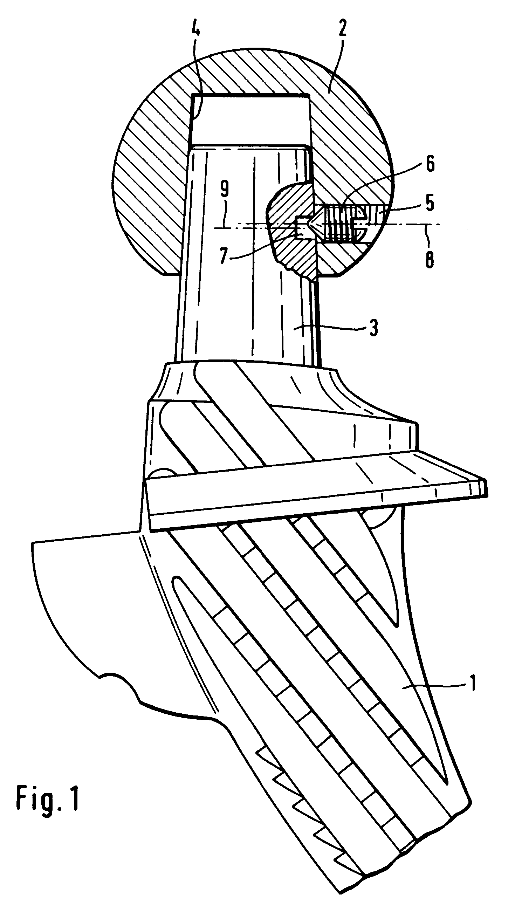

Fig. 1 zeigt einen Teil einer Hüftgelenkendoprothese. Auf den Prothesenhauptteil, den Prothesenschaft 1, der in den Oberschenkelknochen eingesetzt werden soll, soll ein Prothesenkopf 2 aufgesetzt werden. Zu diesem Zweck ist der Prothesenhauptteil 1 im oberen Teil mit einem konusförmigen Vorsprung 3 versehen, während der Prothesenkopf 2 eine entsprechende konusförmige Ausnehmung 4 aufweist. Der Prothesenkopf 2 ist mit einer mit Gewinde versehenen Bohrung 5 versehen, in die eine Sicherungsschraube 6 eingeschraubt werden kann, die vorne kegelförmig in eine Spitze ausläuft. Im konusförmigen Vorsprung 3 ist eine Bohrung 7 vorgesehen, in die die Spitze der Sicherungsschraube eingreifen kann, um so die Konusverbindung der Teile 3, 4 dauerhaft zu fixieren. Wie aus der Fig. ersichtlich ist, ist bei dieser Ausführungsform die Schraube mit ihrer Achse 8 exzentrisch zur Mittellinie zur Achse 9 der Bohrung 7 angeordnet, so daß Schraube 6 und Bohrung 7 sich nur an dem Randbereich berühren, der zum sich verjüngenden Ende des Konus 3 gerichtet ist. Dadurch werden die besonderen Vorteile erzielt, daß die Konusverbindung noch mehr gespannt wird und trotzdem ein weiteres Ineinanderschieben der Teile nicht behindert wird.

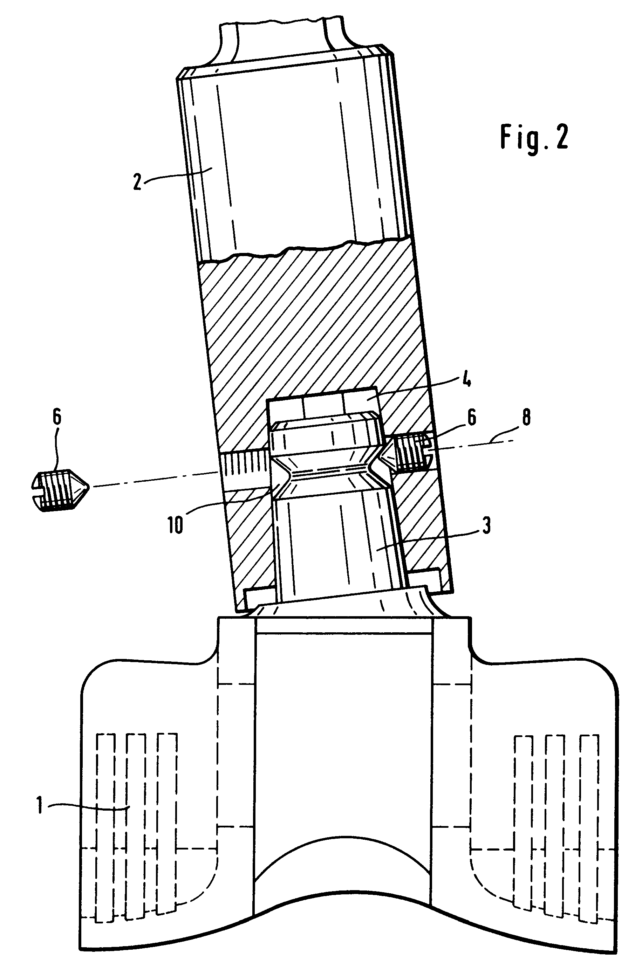

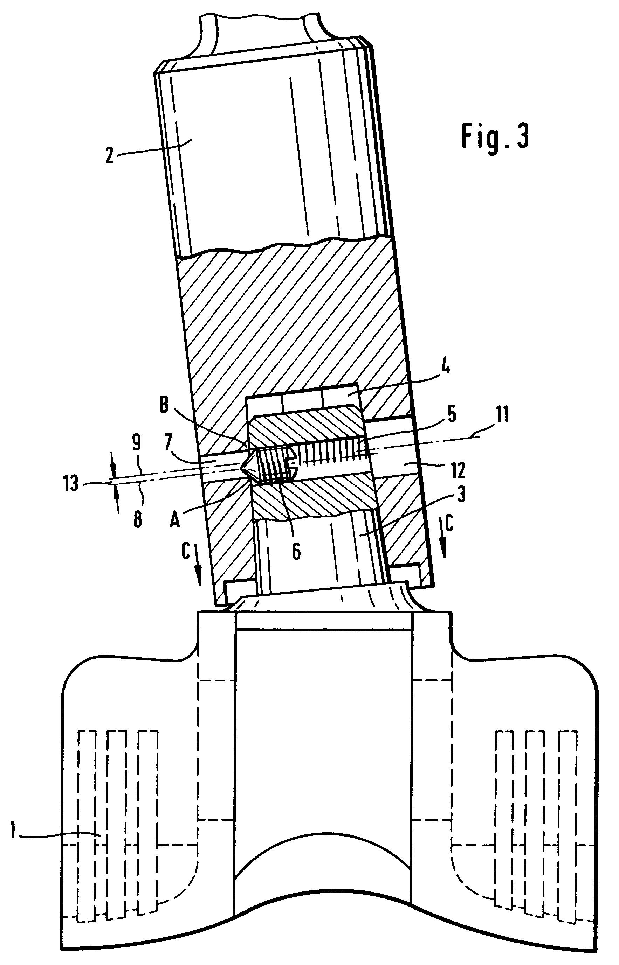

Bei der Ausführungsform der Fig. 2 und 3 ist der Hauptteil 1 ein Kniegelenk, auf das ein Prothesenschaft 2 aufgesetzt werden soll. Statt einer Bohrung 7 ist im konusförmigen Vorsprung 3 eine Ringnut 10 mit V-förmigem Querschnitt vorgesehen, in die von zwei verschiedenen Seiten zwei Schrauben 6 eingreifen können. Wie man deutlich in der Fig. sieht, ist die Ringnut in bezug auf die Schraubenachsen wieder exzentrisch angeordnet, so daß ähnliche Wirkungen wie bei der Ausführungsform der Fig. 1 auftreten. Selbstverständlich müßte die Ringnut nicht symmetrisch sein, da die in der Fig. unteren Flanken der Nut überhaupt nicht mit den Schrauben 6 in Berührung kommen.

Bei der Ausführungsform der Fig. 3 ist die Schraube 6 im inneren Konusteil 3 vorgesehen. Die Schraube wird innerhalb der Gewindebohrung 5 zunächst soweit zurückgeschraubt, daß sie ganz innerhalb des konusförmigen Vorsprunges 3 angeordnet ist. Dann wird der Prothesenschaft 2 auf den Vorsprung 3 geschoben. Anschließend kann durch eine Gegenbohrung 12 dann ein Schraubendreher angesetzt und die Schraube 6 in der Fig. nach links geschraubt werden, so daß sie mit der Bohrung 7, die der Bohrung 12 gegenüberliegt, in Eingriff kommen kann. Auch hier ist die Achse 8 der Schraube 6 exzentrisch oder achsenversetzt zur Achse 9 der Bohrung 7. Der entsprechende Achsenversatz ist mit 13 bezeichnet. Allerdings muß jetzt die Schraube 6 am unteren Rand der Bohrung 7 bei A angreifen, damit der Konusteil 4 fest auf den Konusteil 3 in Richtung der Pfeile C gespannt wird. Am oberen Rand bei B berührt die Sicherungsschraube den Rand der Bohrung 7 nicht, so daß eine Bewegung des Teils 2 in Richtung der Pfeile C, d.h. ein noch festerer Sitz des Teils 2 auf dem Teil 1 nicht behindert wird.

Bei allen Ausführungsformen kann in die mit Gewinde versehene Bohrung 5 nach dem Einschrauben der Schraube 6 noch eine zusätzliche Konterschraube hineingeschraubt werden, die in den Fig. nicht gezeigt ist, um die Verbindung dauerhaft zu fixieren.

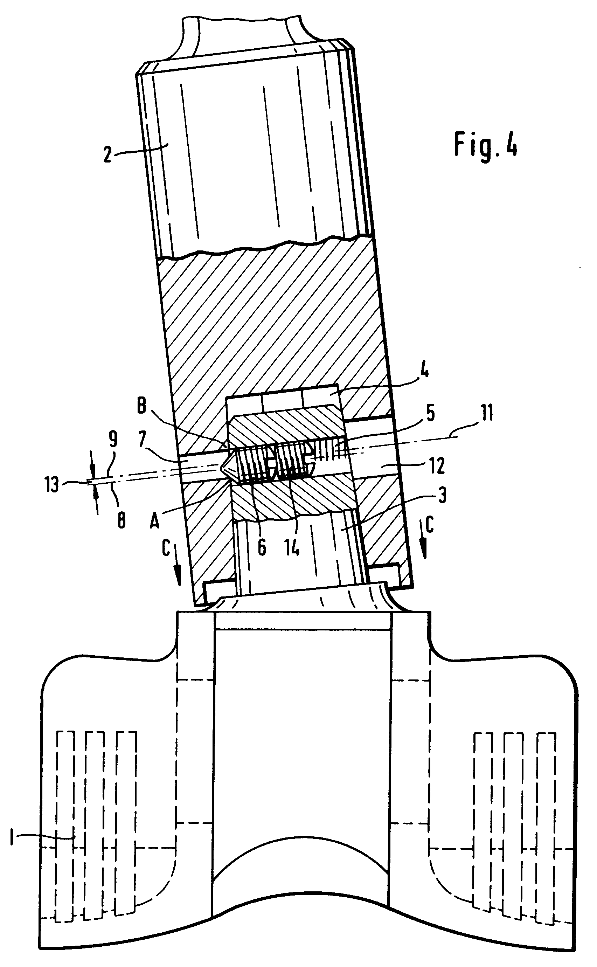

Die Ausführungsform der Fig. 4 entspricht im wesentlichen der Ausführungsform der Fig. 3. Zusätzlich ist dort noch eine Konterschraube 14 hinter der Sicherungsschraube 6 eingeschraubt, um ein Lösen der Sicherungsschraube 6 zuverlässig zu verhindern. Die Konterschraube ist dabei hinter der Sicherungsschraube 6, d.h. auf der Seite angeordnet, die dem vorderen Ende der Sicherungsschraube entgegengesetzt ist, das mit dem anderen Konusteil zusammenwirkt. Solche Sicherungsschrauben 14 können auch bei den Ausführungsformen der Fig. 1 und 2 vorgesehen sein, wobei dann natürlich die Sicherungsschraube 6 kürzer oder der Konusteil, in den sie eingeschraubt ist, dicker sein muß, als dies in den Figuren gezeigt ist.

## Patentansprüche

1. Endoprothese mit zwei oder mehr Einzelteilen, von denen einer ein Konusteil (2) mit einer konusförmigen Bohrung (4) und von denen ein anderer ein Konusteil (1) mit einem komplementären konusförmigen Vorsprung (3) aufweist, wobei die Teile durch Einführen des konusförmigen Vorsprungs (3) in die konusförmige Bohrung (4) verbunden sind und im Bereich der Konusverbindung (3, 4) eine Sicherungsschraube (6) vorgesehen ist, die in einem der Konusteile (1, 2) eingeschraubt ist und die mit ihrem vorderen Ende in eine Ausnehmung (7, 10) im anderen Konusteil (2, 1) eingreift, dadurch gekennzeichnet, daß die Achse (8) der Sicherungsschraube (6) im wesentlichen senkrecht zur Konusachse steht un daß das vordere Ende der Sicherungsschraube (6) kegel- oder kegelstumpfförmig ist und mit der Ausnehmung exzentrisch zusammenwirkt.

2. Endoprothese nach Anspruch 1, dadurch gekennzeichnet, daß die Sicherungsschraube (6) im äußeren Konusteil (2) angeordnet ist und mit einer Bohrung (7) im inneren Konusteil (1) zusammenwirkt.

3. Endoprothese nach Anspruch 1, dadurch gekennzeichnet, daß die Sicherungsschraube (6) im äußeren Konusteil (2) angeordnet ist und mit einer Ringnut (10) im inneren Konusteil (1) zusammenwirkt.

4. Endoprothese nach einem der Ansprüche 1 bis 3, bei der die Sicherungsschraube im äußeren Konusteil angeordnet ist, dadurch gekennzeichnet, daß das vordere Ende der Sicherungsschraube (6) mit der Ausnehmung (7, 10) exzentrisch auf der Seite der Schraubenachse (8) zusammenwirkt, die zum sich verjüngenden Konusende gerichtet ist.

5. Endoprothese nach Anspruch 1, dadurch gekennzeichnet, daß die Sicherungsschraube (6) in einer mit Gewinde versehenen Bohrung (5) des inneren Konusteils (1) angeordnet ist und mit einer Bohrung (7) im äußeren Konusteil zusammenwirkt.

6. Endoprothese nach Anspruch 1, dadurch gekennzeichnet, daß die Sicherungsschraube (6) in einer mit Gewinde versehenen Bohrung (5) des inneren Konusteils (1) angeordnet ist und mit einem sich über einen Teil des Umfangs erstreckenden Schlitz im äußeren Konusteil zusammenwirkt.

7. Endoprothese nach einem der Ansprüche 1, 5 oder 6, bei der die Sicherungsschraube im inneren Konusteil angeordnet ist, dadurch gekennzeichnet, daß das vordere Ende der Sicherungsschraube (6) mit der Ausnehmung (7) exzentrisch auf der Seite der Schraubenachse (8) zusammenwirkt, die vom sich verjüngenden Konusende weggerichtet ist.

8. Endoprothese nach einem der Ansprüche 1 bis 7, dadurch gekennzeichnet, daß die Wände der Ausnehmung (7, 10), mit denen die Sicherungsschraube (6) zusammenwirkt, wenigstens im zur Sicherungsschraube gerichteten Teil abgeschrägt sind.

9. Endoprothese nach Anspruch 8, dadurch gekennzeichnet, daß der Winkel der Abschrägung dem entsprechenden Winkel des kegel- oder kegelstumpfförmigen vorderen Endes der Sicherungsschraube (6) entspricht.

10. Endoprothese nach einem der Ansprüche 1 bis 9, dadurch gekennzeichnet, daß sie eine Konterschraube für die Sicherungsschraube (6) aufweist.

11. Endoprothese nach einem der Ansprüche 1 bis 10, dadurch gekennzeichnet, daß sie mehr als eine Sicherungsschraube (6) aufweist.

12. Endoprothese nach einem der Ansprüche 1 bis 11, dadurch gekennzeichnet, daß hinter der Sicherungsschraube (6) eine Konterschraube (14) angeordnet ist.

## Claims

1. Endoprosthesis with two or more individual parts, whereof one comprises a cone part (2) with a conical bore (4) and whereof another comprises a cone part (1) with a complementary conical projection (3), the parts being connected by introducing the conical projection (3) into the conical bore (4) and provided in the region of the cone connection (3, 4) is a locking screw (6), which is screwed into one of the cone parts (1, 2) and engages by its front end in a recess (7, 10) in the other cone part (2, 1), characterised in that the axis (8) of the locking screw (6) is substantially perpendicular to the cone axis and that the front end of the locking screw (6) is conical or frustoconical and cooperates eccentrically with the recess.

2. Endoprosthesis according to Claim 1, characterised in that the locking screw (6) is located in the outer cone part (2) and cooperates with a bore (7) in the inner cone part (1).

3. Endoprosthesis according to Claim 1, characterised in that the locking screw (6) is located in the outer cone part (2) and cooperates with an annular groove (10) in the inner cone part (1).

4. Endoprosthesis according to one of Claims 1 to 3, in which the locking screw is located in the outer cone part, characterised in that the front end of the locking screw (6) cooperates with the recess (7, 10) eccentrically on the side of the screw axis (8), which is directed towards the tapering cone end.

5. Endoprosthesis according to Claim 1, characterised in that the locking screw (6) is located in a bore (5) of the inner cone part (1) provided with a screw thread and cooperates with a bore (7) in the outer cone part.

6. Endoprosthesis according to Claim 1, characterised in that the locking screw (6) is located in a bore (5) of the inner cone part (1) provided with a screw thread and cooperates with a slot in the outer cone part extending over part of the periphery.

7. Endoprosthesis according to one of Claims 1, 5 or 6, in which the locking screw is located in the inner cone part, characterised in that the front end of the locking screw (6) cooperates with the recess (7) eccentrically on the side of the screw axis (8), which is directed away from the tapering cone end.

8. Endoprosthesis according to one of Claims 1 to 7, characterised in that the walls of the recess (7, 10), with which the locking screw (6) cooperates, are bevelled at least in the part directed towards the locking screw.

9. Endoprosthesis according to Claim 8, characterised in that the angle of the bevel corresponds to the corresponding angle of the conical or frustoconical front end of the locking screw (6).

10. Endoprosthesis according to one of Claims 1 to 9, characterised in that it comprises a counter-screw for the locking screw (6).

11. Endoprosthesis according to one of Claims 1 to 10, characterised in that it comprises more than one locking screw (6).

12. Endoprosthesis according to one of Claims 1 to 11, characterised in that a counter-screw (14) is located behind the locking screw (6).

## Revendications

1. Endoprothèse en deux ou plusieurs parties séparées, dont l'une présente une partie conique (2) avec une forure conique (4) et dont une autre présente une partie conique (1) avec une saillie conique complémentaire (3), ces parties étant assemblées par insertion de la saillie conique (3) dans la forure conique (4) et une vis de blocage (6), prévue dans la région de l'assemblage conique (3, 4), étant vissée dans l'une des parties coniques (1, 2) et s'engageant, par son extrémité avant, dans un creux (7, 10) dans l'autre partie conique (2, 1), caractérisée en ce que l'axe (8) de la vis de blocage (6) est essentiellement perpendiculaire à l'axe du cône, et en ce que l'extrémité avant de la vis de blocage (6) est conique ou tronconique et coopère excentriquement avec le creux.

2. Endoprothèse selon la revendication 1, caractérisée en ce que la vis de blocage (6) est disposée dans la partie conique extérieure (2) et coopère avec une forure (7) dans la partie conique intérieure (1).

3. Endoprothèse selon la revendication 1, caractérisée en ce que la vis de blocage (6) est disposée dans la partie conique extérieure (2) et coopère avec une gorge annulaire (10) dans la partie conique intérieure (1).

4. Endoprothèse selon l'une quelconque des revendications 1 à 3, dans laquelle la vis de blocage est disposée dans la partie conique extérieure, caractérisée en ce que l'extrémité avant de la vis de blocage (6) coopère excentriquement avec le creux (7, 10) du côté de l'axe (8) de la vis qui est situé vers l'extrémité de section réduite du cône d'assemblage.

5. Endoprothèse selon la revendication 1, caractérisée en ce que la vis de blocage (6) est disposée dans une forure taraudée (5) de la partie conique intérieure (1) et coopère avec une forure (7) dans la partie conique extérieure.

6. Endoprothèse selon la revendication 1, caractérisée en ce que la vis de blocage (6) est disposée dans une forure taraudée (5) de la partie conique intérieure (1) et coopère avec une rainure qui s'étend sur une partie de la périphérie dans la partie conique extérieure.

7. Endoprothèse selon l'une quelconque des revendications 1, 5 ou 6, dans laquelle la vis de blocage est disposée dans la partie conique intérieure, caractérisée en ce que l'extrémité avant de la vis de blocage (6) coopère excentriquement avec le creux (7) du côté de l'axe (8) de la vis qui est situé à l'opposé de l'extrémité de section réduite du cône d'assemblage.

8. Endoprothèse selon l'une quelconque des revendications 1 à 7, caractérisée en ce que les parois du creux (7, 10) avec lesquelles coopère la vis de blocage (6) sont biseautées, au moins dans la partie dirigée vers la vis de blocage.

9. Endoprothèse selon la revendication 8, caractérisée en ce que l'angle du biseau correspond à l'angle correspondant de l'extrémité avant conique ou tronconique de la vis de blocage (6).

10. Endoprothèse selon l'une quelconque des revendications 1 à 9, caractérisée en ce qu'elle présente une contre-vis de sécurité pour la vis de blocage (6).

11. Endoprothèse selon l'une quelconque des revendications 1 à 10, caractérisée en ce qu'elle présente plus d'une vis de blocage (6).

12. Endoprothèse selon l'une quelconque des revendications 1 à 11, caractérisée en ce qu'une contre-vis de sécurité (14) est disposée en arrière de la vis de blocage (6).
